(19) Europäisches Patentamt
European Patent Office
Office européen des brevets

(11) Veröffentlichungsnummer: **0 145 907 B1**

(12) **EUROPÄISCHE PATENTSCHRIFT**

(45) Veröffentlichungstag der Patentschrift: **11.05.88**

(51) Int. Cl.⁴: **C 07 C 121/76**

(21) Anmeldenummer: **84113033.9**

(22) Anmeldetag: **30.10.84**

(54) **Verfahren zur Herstellung von alpha-Ketonitrilen.**

(30) Priorität: **11.11.83 DE 3340933**

(43) Veröffentlichungstag der Anmeldung: **26.06.85 Patentblatt 85/26**

(45) Bekanntmachung des Hinweises auf die Patenterteilung: **11.05.88 Patentblatt 88/19**

(84) Benannte Vertragsstaaten: **BE CH DE FR GB IT LI NL**

(56) Entgegenhaltungen:
**BULLETIN DE LA SOCIETE CHIMIQUE DE FRANCE, fascicule 6, Juni 1970, Seiten 2309-2322; C. KATAMNA: "Synthèse des aryl-2 benzofurannones-3"**
**THE JOURNAL OF ORGANIC CHEMISTRY, Band 28, Nr. 4, April 1963, Seiten 1075-1079; Y. OKUMURA: "Abnormal reaction of benzoin with thionyl chloride"**

(73) Patentinhaber: **BAYER AG, Konzernverwaltung RP Patentabteilung, D-5090 Leverkusen 1 Bayerwerk (DE)**

(72) Erfinder: **Kranz, Eckart, Dr., Am Acker 9, D-5600 Wuppertal 1 (DE)**
Erfinder: **Findeisen, Kurt, Dr., In der Follmühle 10, D-5068 Odenthal 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 145 907 B1

ACTORUM AG

**Beschreibung**

Die vorliegende Erfindung betrifft ein chemisch eigenartiges Verfahren zur Herstellung von α-Ketonitrilen.

Es ist bekannt, α-Ketonitrile aus Carbonsäurehalogeniden zu erzeugen, indem diese in Gegenwart katalytischer Mengen von Kupfer(I)-cyanid, Kupfer(II)-cyanid, Zinkcyanid oder deren Komplexverbindungen mit Alkalicyaniden oder Cyanwasserstoff bei Temperaturen von 100°C bis 300°C umgesetzt werden (vgl. z.B. DE-AS 2 614 242).

Ausserdem ist bekannt, dass bei der Umsetzung von Cyanhydrinen mit Thionylchlorid eine Substitution der Hydroxy-Gruppierung stattfindet (vgl. dazu J. Chem. Soc. 1945, 352; JP 57 123 175; Derwent Referatnummer 69 071 R der SU-PS 255 294 und Derwent Referatnummer 27 727 S der SU-PS 268 438). Diese Substitution tritt ebenso auf bei der Reaktion von Alkoholen mit Sulfurylchlorid (vgl. dazu Houben-Weyl-Müller, Band IV/2, S. 466, Thieme Verlag Stuttgart).

Überraschenderweise wurde nun gefunden, dass man α-Ketonitrile der Formel (I)

$$R-\overset{O}{\overset{\|}{C}}-C\equiv N \qquad (I)$$

in welcher

R für gegebenenfalls substituiertes Aryl steht,

erhält, wenn man Cyanhydrine der Formel (II)

$$R-\overset{OH}{\overset{|}{C}}H-CN \qquad (II)$$

in welcher

R die oben angegebene Bedeutung hat,

mit Sulfurylchlorid oder Thionylchlorid, gegebenenfalls in Gegenwart von chlorierten Kohlenwasserstoffen bei Temperaturen zwischen 15°C und 30°C umsetzt.

Überraschenderweise gelingt es, mit Hilfe des erfindungsgemässen Verfahrens eine Oxidation der Cyanhydrine durchzuführen. Dies ist umso überraschender, als nach dem Stand der Technik bei der Umsetzung von Cyanhydrinen mit Sulfurylchlorid oder Thionylchlorid entweder die Bildung von α-Chlorsulfonylnitrilen bzw. α-Chlorsulfinylnitrilen oder α-Chlornitrilen in beträchtlichem Ausmass hätte erwartet werden müssen (vgl. dazu US-PS 3 470 493; Derwent Referatnummer 69 071 R der SU-PS 255 294 und Derwent Referatnummer 27 727 S der SU-PS 268 438).

Bevorzugt wird das erfindungsgemässe Verfahren verwendet für die Herstellung der folgenden α-Ketonitrile:

2-Chlor-, 4-Chlor-, 3,4-Dichlor-, 3,5-Dichlor-, 3,4,5-Trichlor-, 2,6-Dichlor- und 2,4-Dichlor-benzoylcyanid und Benzoylcyanid.

Besonders bevorzugt wird das erfindungsgemässe Verfahren verwendet für die folgenden Verbindungen der Formel (I):

2-Chlor-, 4-Chlor-, 3,4-Dichlor-, 3,5-Dichlor- und 3,4,5-Trichlor-benzoylcyanid.

Verwendet man 3,4-Dichlor-α-hydroxy-benzylnitril und Sulfurylchlorid als Ausgangsstoffe, so kann der Reaktionsablauf durch das folgende Formelschema (a) wiedergegeben werden:

(a) 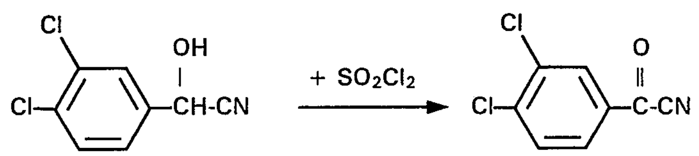


Verwendet man 4-Chlor-α-hydroxy-benzylnitril und Thionylchlorid als Ausgangsstoffe, so kann der Reaktionsablauf durch das folgene Formelschema (b) wiedergegeben werden:

(b) 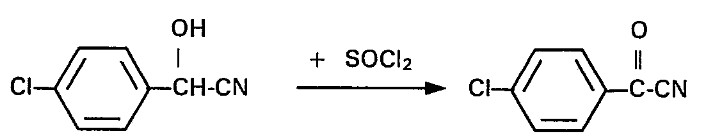


Die als Ausgangsstoffe verwendeten Cyanhydrine sind durch die Formel (II) allgemein definiert. In dieser Formel steht R vorzugsweise für gegebenenfalls einfach bis dreifach durch $C_1$-$C_4$-Alkyl, $C_1$-$C_4$-Alkoxy, Nitro und/oder Halogen, wie Fluor, Chlor oder Brom, substituiertes Aryl, wie insbesondere Phenyl.

Als bevorzugte Beispiele für Cyanhydrine der Formel (II) seien im einzelnen genannt: Phenyl-, 2-Chlorphenyl-, 4-Chlorphenyl-, 3,4-Dichlorphenyl-, 3,5-Dichlorphenyl-, 2,4-Dichlorphenyl-, 4-Methoxyphenyl- und 3,4,5-Trichlorphenyl-cyano-hydroxy-methan.

Die Cyanhydrine der Formel (II) sind allgemein bekannte Verbindungen der organischen Chemie.

Als Verdünnungsmittel, die bei der Durchführung des erfindungsgemässen Verfahrens eingesetzt werden können, kommen vorzugsweise halogenierte Kohlenwasserstoffe, wie Chloroform, Methylenchlorid, Methylchlorid und Tetrachlorkohlenstoff in Betracht. Prinzipiell ist es jedoch auch möglich, die erfindungsgemässe Umsetzung ohne Verdünnungsmittel durchzuführen.

Die Reaktion wird im allgemeinen bei Temperaturen zwischen 15°C und 30°C, vorzugsweise bei Raumtemperatur durchgeführt.

Die Reaktion wird im allgemeinen bei Normaldruck durchgeführt.

Bei der Durchführung des erfindungsgemässen Verfahrens setzt man im allgemeinen auf 1 Mol Cyanhydrin der Formel (II) 1,0 bis 10,0 Mol, vorzugsweise 1,0 bis 2,5 Mol Sulfurylchlorid oder Thionylchlorid ein.

Die Aufarbeitung erfolgt nach üblichen Methoden.

Die Reinheit der Verbindungen wird gaschromatographisch ermittelt. Zu ihrer Charakterisierung dienen GC-MS-Daten und/oder der Schmelzpunkt.

Die nach dem Verfahren leicht zugänglichen α-Ketonitrile lassen sich beispielsweise zur Synthese von Insektiziden einsetzen. So lassen sich z.B. aus ihnen substituierte Hydroxymalonsäurediamide, Verbindungen mit hoher insektizider Potenz, z.B. gemäss den folgenden Gleichungen herstellen:

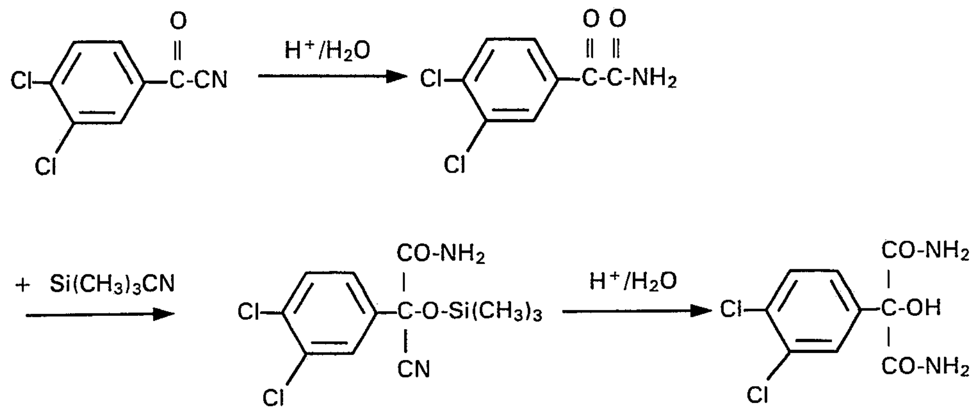


Die substituierten Hydroxymalonsäurediamide und ihre Verwendung als Insektizide sind bekannt (vgl. DE-OS 3 140 275).

*Herstellungsbeispiele:*

*Beispiel 1a*

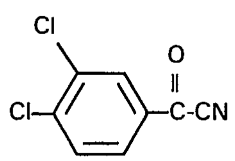

20,2 g (0,1 Mol) Cyano-(3,4-dichlorphenyl)-(hydroxy)-methan werden in 100 ml Methylenchlorid gelöst. Anschliessend werden 13,1 g (0,11 Mol) Sulfurylchlorid bei Raumtemperatur zugetropft und 16 Stunden bei dieser Temperatur nachgerührt. Der geringfügig entstandene Niederschlag wird abgesaugt und die Lösung wird vom Lösungsmittel und überschüssigem Sulfurylchlorid befreit.

Man erhält 21,3 g eines öligen Produktes, das langsam auskristallisiert. Der GC-Gehalt an 3,4-Dichlorbenzoylcyanid beträgt 85,9%. Die Ausbeute entspricht 91,2% der Theorie.

Bei der Destillation im Hochvakuum (Kp. 0,2 mbar/112°C erhält man reines 3,4-Dichlorbenzoylcyanid (GC-Gehalt 97%ig) mit dem Schmelzpunkt 70°C bis 71°C.

*Beispiel 1b*

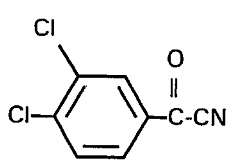

20,2 g (0,1 Mol) Cyano-(3,4-dichlorphenyl)-(hydroxy)-methan werden in 50 ml (81,9 g/0,69 Mol) Thionylchlorid bei Raumtemperatur eingetragen. Man lässt 24 Stunden bei dieser Temperatur nachrühren, saugt den geringfügig entstandenen Niederschlag ab und befreit das Reaktionsprodukt vom überschüssigen Thionylchlorid.

Man erhält 19,4 g eines öligen Produktes. Der GC-Gehalt an 3,4-Dichlorbenzoylcyanid beträgt 51,5%; die Ausbeute entspricht 50,5% der Theorie.

Die Aufreinigung erfolgt über eine Hochvakuumdestillation (113°C bis 114°C/0,3 mbar). Das erhaltene reine 3,4-Dichlorbenzoylcyanid hat einen Schmelzpunkt von 70°C bis 71°C.

Analog Beispiel (1a) und (1b) können die folgenden Verbindungen der Formel (I) erhalten werden:

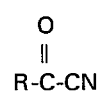 (I)

| Beispiel Nr. | R | Ausbeute (% der Theorie) | Schmelzpunkt (°C) |
|---|---|---|---|
| 2 | 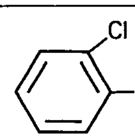 | 83,2 | 35 |
| 3 | 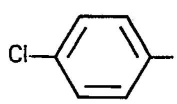 | 82,2 | 40 - 41 |
| 4 | 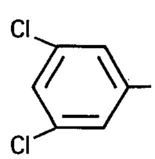 | 80,0 | |

| Beispiel Nr. | R | Ausbeute (% der Theorie) | Schmelzpunkt (°C) |
|---|---|---|---|
| 5 | 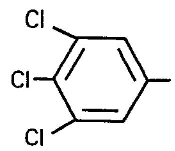 | 89,2 | |
| 6 | 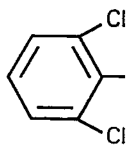 | 40,0 | |
| 7 | 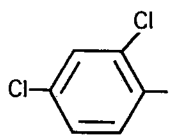 | 12,5 | |
| 8 | 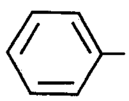 | 36,4 | 31 |

*Vergleichsbeispiel (A)*

$$\begin{array}{l} \quad CH_3 \\ \quad | \\ CH_3\text{-}C\text{-}CH\text{-}O\text{-}SO_2Cl \\ \quad | \quad\; | \\ \quad CH_3 \;\; C{\equiv}N \end{array}$$

11,3 g (0,1 Mol) Cyano-(2,2-dimethyl)-(1-hydroxy)-propan werden in 20,2 ml (33,7 g/0,25 Mol) Sulfurylchlorid 6 Stunden bei 60°C verrührt. Man befreit vom überschüssigen Sulfurylchlorid und erhält 20,2 g (95% Rohausbeute) eines öligen Produktes. Der GC-Gehalt an Cyan-(2,2-dimethyl)-(1-chlorsulfonyl)-propan beträgt 84,4%. Bei der Destillation im Wasserstrahlvakuum erhält man bei 98°C bis 99°C/20 mbar reines Produkt.

**Patentansprüche**

1. Verfahren zur Herstellung von α-Ketonitrilen der Formel (I)

$$\begin{array}{c} O \\ \| \\ R\text{-}C\text{-}C{\equiv}N \end{array} \qquad (I)$$

in welcher
R für gegebenenfalls substituiertes Aryl steht, dadurch gekennzeichnet, dass man Cyanhydrine der Formel (II)

$$\begin{array}{c} OH \\ | \\ R\text{-}CH\text{-}CN \end{array} \qquad (II)$$

in welcher
R die oben angegebene Bedeutung hat, mit Sulfurylchlorid oder Thionylchlorid, gegebenenfalls in Gegenwart von chlorierten Kohlenwasserstoffen bei Temperaturen zwischen 15°C und 30°C umsetzt.

2. Verfahren gemäss Anspruch 1, dadurch gekennzeichnet, dass R für gegebenenfalls einfach bis dreifach durch $C_1$-$C_4$-Alkyl, $C_1$-$C_4$-Alkoxy, Nitro und/oder Halogen, wie Fluor, Chlor oder Brom, substituiertes Phenyl steht.

3. Verfahren gemäss Anspruch 1, dadurch gekennzeichnet, dass R für Phenyl, 2-Chlorphenyl, 4-Chlorphenyl, 3,4-Dichlorphenyl, 3,5-Dichlorphenyl, 2,4-Dichlorphenyl, 2,6-Dichlorphenyl, 4-Methoxyphenyl und 3,4,5-Trichlorphenyl steht.

**Claims**

1. Process for preparing α-ketonitriles of the formula (I)

$$\begin{array}{c} O \\ \| \\ R\text{-}C\text{-}C{\equiv}N \end{array} \qquad (I)$$

in which
R represents optionally substituted aryl, characterised in that cyanohydrins of the formula (II)

$$\begin{array}{c} OH \\ | \\ R\text{-}CH\text{-}CN \end{array} \qquad (II)$$

in which
R has the abovementioned meaning, are reacted at temperatures between 15°C and 30°C with sulphuryl chloride or thionyl chloride in the presence or absence of chlorinated hydrocarbons.

2. Process according to claim 1, characterised in that R represents phenyl which is optionally monosubtituted to trisubstituted by $C_1$-$C_4$-alkyl, $C_1$-$C_4$-alkoxy, nitro and/or halogen, such as fluorine, chlorine or bromine.

3. Process according to claim 1, characterised in that R represents phenyl, 2-chlorophenyl, 4-chlorophenyl, 3,4-dichlorophenyl, 3,5-dichlorophenyl, 2,4-dichlorophenyl, 2,6-dichlorophenyl, 4-methoxyphenyl and 3,4,5-trichlorphenyl.

**Revendications**

1. Procédé de préparation d'α-cétonitriles de formule (I)

$$\begin{array}{c} O \\ \| \\ R\text{-}C\text{-}C{\equiv}N \end{array} \qquad (I)$$

dans laquelle
R représente un aryle éventuellement substitué, caractérisé en ce qu'on fait réagir des cyanhydrines de formule (II)

$$\begin{array}{c} OH \\ | \\ R\text{-}CH\text{-}CN \end{array} \qquad (II)$$

dans laquelle R a la signification indiquée plus haut, avec du chlorure de sulfuryle ou du chlorure de tionyle, éventuellement en présence d'hydrocarbures chlorés à des températures entre 15°C et 30°C.

2. Procédé selon la revendication 1, caractérisé en ce que R est un phényle éventuellement substitué une à trois fois par un alcoyle en $C_1$-$C_4$, alcoxy en $C_1$-$C_4$, nitro et/ou halogène comme le fluor, le chlore ou le brome.

3. Procédé selon la revendication 1, caractérisé en ce que R représente un phényle, 2-chlorophényle, 4-chlorophényle, 3,4-dichlorophényle, 3,5-dichlorophényle, 2,4-dichlorophényle, 2,6-dichlorophényle, 4-méthoxyphényle et 3,4,5-trichlorophényle.